# EUROPEAN PATENT SPECIFICATION

(11) **EP 2 721 990 B1**
(45) Date of publication and mention of the grant of the patent: **11.01.2017**
(21) Application number: 12799937.3
(22) Date of filing: 29.05.2012
(51) Int. Cl.: A61B 1/00, A61B 1/04, A61B 5/07

(54) **IN-VIVO INFORMATION ACQUISITION SYSTEM AND METHOD FOR CONTROLLING AN IN-VIVO INFORMATION ACQUISITION SYSTEM**
IN-VIVO-INFORMATIONSBESCHAFFUNGSSYSTEM UND VERFAHREN ZUM STEUERN EINES IN-VIVO-INFORMATIONSBESCHAFFUNGSSYSTEMS
SYSTÈME IN-VIVO D'ACQUISITION D'INFORMATIONS ET PROCÉDÉ DE COMMANDE POUR UN SYSTÈME IN VIVO D'ACQUISITION D'INFORMATION

(30) Priority: 16.06.2011 JP 2011134396
(43) Date of publication of application: 23.04.2014
(73) Proprietor: Olympus Corporation, Tokyo 192-8507 (JP)
(72) Inventor: MIYAHARA Hideharu, Hachioji-shi Tokyo 192-8507 (JP); MIYATA Kenji, Hachioji-shi Tokyo 192-8507 (JP)
(74) Representative: Winter, Brandl, Fürniss, Hübner, Röss, Kaiser, Polte - Partnerschaft mbB
(86) International application number: PCT/JP2012/063714
(87) International publication number: WO 2012/172964

(56) References cited:
- EP-A1- 1 815 781
- JP-A- 2003 070 728
- JP-A- 2003 179 647
- JP-A- 2005 152 401
- JP-A- 2005 342 083
- JP-A- 2008 278 355
- US-A1- 2005 043 634
- US-A1- 2005 195 785

## Description

### Technical Field

The present invention relates to an in-vivo information acquisition system, and more particularly, to an in-vivo information acquisition system capable of acquiring in-vivo information and a method for controlling the in-vivo information acquisition system.

### Background Art

Endoscopes in a medical field have been conventionally used for the purpose of in-vivo observation and the like. As one kind of the above endoscopes, a capsule endoscope has been proposed in recent years which is arranged in a body cavity by being swallowed by a subject, can pick up images of an object while moving through the body cavity accompanying vermiculation, and wirelessly transmit the picked-up images of the object to an outside as image-pickup signals.

As a capsule endoscope having a configuration substantially similar to the capsule endoscope described above, for example, the capsule endoscope disclosed in Japanese Patent Application Laid-Open Publication No. 2005-342083 is given.

More specifically, Japanese Patent Application Laid-Open Publication No. 2005-342083 discloses a configuration of a system including a capsule-type medical apparatus acquiring in-vivo information of a subject and an in-vivo information receiving apparatus receiving the in-vivo information, wherein a communication confirmation signal for confirming condition of communication with the in-vivo information receiving apparatus is transmitted from the capsule-type medical apparatus; a communication permission signal for permitting communication is transmitted from the in-vivo information receiving apparatus when the communication confirmation signal is received; and the in-vivo information is transmitted from the capsule-type medical apparatus when the communication permission signal is received.

However, according to the configuration disclosed in Japanese Patent Application Laid-Open Publication No. 2005-342083, a problem occurs that, because operations related to transmission and reception of the signal for confirming the condition of communication are operated before the in-vivo information obtained by the capsule-type medical apparatus is transmitted to the in-vivo information receiving apparatus, control related to communication operations is complicated, and power consumption increases.

Document US 2005/0043634 A1 describes a capsule type medical apparatus communication system which includes a capsule type medical apparatus and an information receiver, wherein the capsule type medical apparatus transmits towards the information receiver a communication confirmation signal which confirms the state of communication with the information receiver, while the information receiver, when it has received the communication confirmation signal, transmits a communication authorization signal which authorizes the capsule type medical apparatus to perform communication. The capsule type medical apparatus further includes a communication control device which transmits the information when it has received the communication authorization signal.

The present invention has been made in view of the situation described above, and its object is to provide an in-vivo information acquisition system capable of simplifying the control related to communication operations in comparison with conventional systems as well as reducing power consumed at the time of the communication operations in comparison with the conventional systems, and a method for controlling the in-vivo information acquisition system. The object is solved by the features of the independent claims. The dependent claims are directed to preferred embodiments of the invention.

### Disclosure of Invention

### Means for Solving the Problem

An in-vivo information acquisition system of an aspect of the present invention is an in-vivo information acquisition system including an in-vivo information acquisition apparatus provided with a configuration capable of transmitting a wireless signal which includes in-vivo information about an inside of a subject acquired by an in-vivo information acquisition section to an outside of the subject, and a terminal apparatus provided with a configuration capable of receiving the wireless signal transmitted from the in-vivo information acquisition apparatus outside the subject, wherein the terminal apparatus includes a reception confirmation signal transmission section generating a reception confirmation signal and transmitting the reception confirmation signal to the in-vivo information acquisition apparatus when the wireless signal which includes the in-vivo information about the subject can be received; and the in-vivo information acquisition apparatus includes a storage section, and a control section causing the wireless signal which includes the in-vivo information about the subject to be transmitted to the outside of the subject at the same time, causing the same in-vivo information as included in the wireless signal transmitted to the outside of the subject to be stored into the storage section at least during a period during which the reception confirmation signal transmitted from the terminal apparatus cannot be received.

An in-vivo information acquisition system control method of an aspect of the present invention is a method for controlling an in-vivo information acquisition system including an in-vivo information acquisition apparatus provided with a configuration capable of transmitting a wireless signal which includes in-vivo information about an inside of a subject acquired by an in-vivo information acquisition section to an outside of the subject, and a terminal apparatus provided with a configuration capable of receiving the wireless signal transmitted from the in-vivo information acquisition apparatus outside the subject, the method including the steps of: a reception confirmation signal being generated and transmitted to the in-vivo information acquisition apparatus by an operation of a reception confirmation signal transmission section of the terminal apparatus when the wireless signal which includes the in-vivo information about the subject is received; the wireless signal which includes the in-vivo information about the subject being transmitted to the outside of the subject by an operation of a control section of the in-vivo information acquisition apparatus at least during a period during which the reception confirmation signal transmitted from the terminal apparatus cannot be received; and the same in-vivo information as included in the wireless signal transmitted to the outside of the subject being stored into a storage section by an operation of the control section at least during the period during which the reception confirmation signal transmitted from the terminal apparatus cannot be received.

### Brief Description of the Drawings

Fig. 1 is a diagram showing a configuration of main parts of an in-vivo information acquisition system in a first embodiment of the present invention;
Fig. 2 is a schematic diagram for illustrating an aspect of use of the in-vivo information acquisition system;
Fig. 3 is a timing chart for illustrating operations of an in-vivo information acquisition apparatus and a terminal apparatus in the first embodiment; and
Fig. 4 is a timing chart for illustrating operations of an in-vivo information acquisition apparatus and a terminal apparatus in a second embodiment.

### Best Mode for Carrying Out the Invention

Embodiments of the present invention will be described below with reference to drawings.

### (First Embodiment)

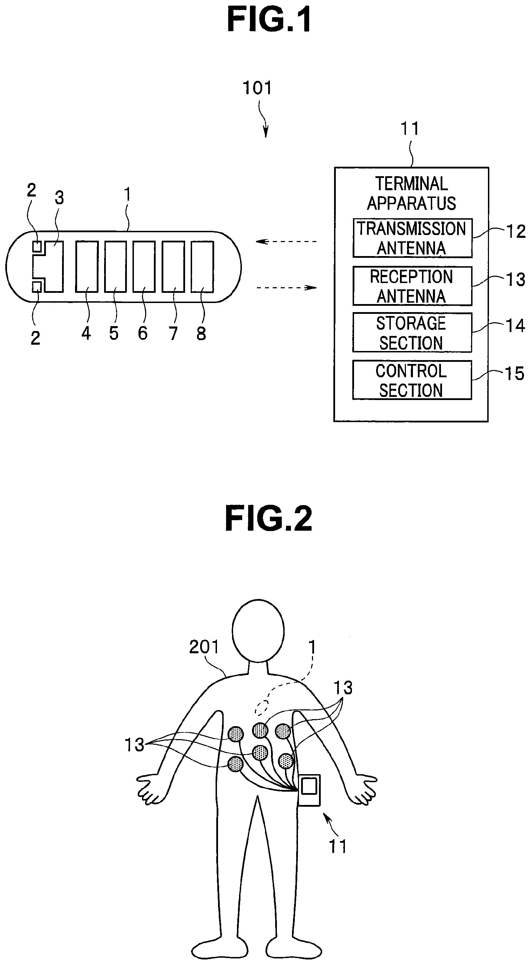

Figs. 1 to 3 relate to a first embodiment of the present invention.

Fig. 1 is a diagram showing a configuration of main parts of an in-vivo information acquisition system in the first embodiment of the present invention.

An in-vivo information acquisition system 101 is configured being provided with an in-vivo information acquisition apparatus 1 and a terminal apparatus 11 as shown in Fig. 1.

The in-vivo information acquisition apparatus 1 is configured as an apparatus having a size, shape and the like which can be arranged in a body cavity of a subject, for example, like a capsule endoscope.

The in-vivo information acquisition apparatus 1 is configured being provided with an illumination section 2 emitting illuminating light for illuminating an object in the body cavity of the subject, an image pickup section 3 picking up the object illuminated by the illumination section 2 and acquiring image data, a wireless transmission section 4 modulating the image data acquired by the image pickup section 3 to a wireless signal and transmitting the wireless signal to the terminal apparatus 11, a wireless reception section 5 receiving the wireless signal transmitted from the terminal apparatus 11 and demodulating the wireless signal, a storage section 6 capable of accumulating image data acquired by the image pickup section 3, a power source section 7 capable of supplying drive power required for driving each section of the in-vivo information acquisition apparatus 1, and a control section 8 performing control related to operations of each section of the in-vivo information acquisition apparatus 1.

That is, an in-vivo information acquisition section in the present embodiment is configured being provided with the illumination section 2 and the image pickup section 3.

The terminal apparatus 11 is configured as a portable apparatus which can be driven by power supplied from an internal battery not shown, for example, like a mobile terminal apparatus.

The terminal apparatus 11 is configured being provided with a transmission antenna 12 modulating a reception conformation signal to be described later to a wireless signal and transmitting the wireless signal to the in-vivo information acquisition apparatus 1, a reception antenna 13 receiving the wireless signal transmitted from the in-vivo information acquisition apparatus 1 and demodulating the wireless signal, a storage section 14 capable of accumulating image data received by the reception antenna 13, and a control section 15 performing control related to operations of each section of the terminal apparatus 11 including generation of a reception confirmation signal to be described later.

That is, a reception confirmation signal transmission section in the present embodiment is configured being provided with the transmission antenna 12 and the control section 15.

Note that the in-vivo information acquisition apparatus 1 in the present embodiment is not limited to one configured so as to operate in response to drive power supplied from the power source section 7, which is an internal battery or the like. For example, the in-vivo information acquisition apparatus 1 may be configured so as to operate in response to drive power supplied from the outside by wireless power supply utilizing an electromagnetic phenomenon.

Fig. 2 is a schematic diagram for illustrating an aspect of use of the in-vivo information acquisition system.

The in-vivo information acquisition system 101 of the present embodiment is used for a subject 201, for example, according to an aspect shown in Fig. 2. More specifically, at the time of using the in-vivo information acquisition system 101, the in-vivo information acquisition apparatus 1 is arranged in the body cavity of the subject 201; a body part of the terminal apparatus 11 is fixed to the outside of the body of the subject 201; and a plurality of the reception antennas 13 connected via signal lines extending from the body part of the terminal apparatus 11, respectively, are arranged on a body surface part of the subject 201.

Note that the body part of the terminal apparatus 11 is not limited to one configured so as to be usable in a state of being fixed to the subject 201. The body part of the terminal apparatus 11 may be configured as such that is on the assumption of being used in a state of being set at a predetermined position, for example, like a workstation.

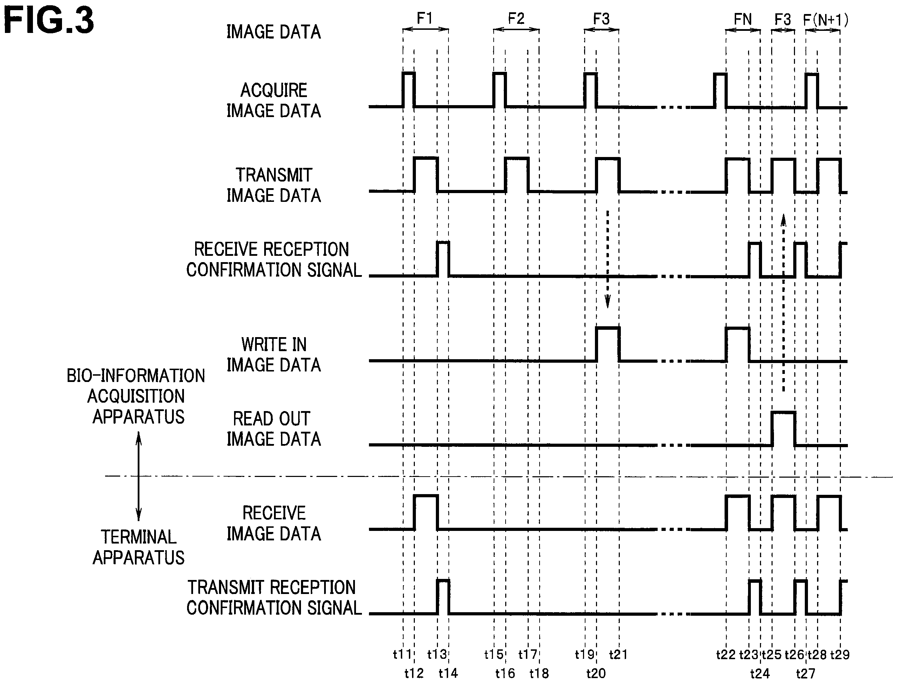

Here, specific operations of the in-vivo information acquisition apparatus 1 and the terminal apparatus 11 in the present embodiment will be described with appropriate reference to a timing chart in Fig. 3. Fig. 3 is a timing chart for illustrating operations of the in-vivo information acquisition apparatus and the terminal apparatus in the first embodiment.

First, at arbitrary timing before time t11, each of power sources for the in-vivo information acquisition apparatus 1 and the terminal apparatus 11 is on, and each of the in-vivo information acquisition apparatus 1 and the terminal apparatus 11 is arranged so that the in-vivo information acquisition apparatus 1 and the terminal apparatus 11 have a positional relationship illustrated in Fig. 2.

After that, the control section 8 of the in-vivo information acquisition apparatus 1 performs control for causing the image pickup section 3 to pick up an image of an object in the body cavity of the subject 201 to acquire image data F1 during a period from the time t11 to time t12. Furthermore, the control section 8 of the in-vivo information acquisition apparatus 1 performs control for causing the image data F1 acquired by the image pickup section 3 to be transmitted from the wireless transmission section 4 to the terminal apparatus 11 during a period from the time t12 to time t13.

If a wireless signal which includes the image data F1 can be received by the reception antennas 13 during the period from the time t12 to the time t13, the control section 15 of the terminal apparatus 11 causes the image data F1 to be stored into the storage section 14. Furthermore, the control section 15 of the terminal apparatus 11 performs control for causing a reception confirmation signal indicating that the wireless signal which includes the image data F1 could be received to be generated and transmitted from the transmission antenna 12 to the in-vivo information acquisition apparatus 1 during a period from the time t13 to time t14. Then, immediately after transmission of the reception confirmation signal indicating that the wireless signal which includes the image data F1 could be received is completed, the control section 15 of the terminal apparatus 11 transitions to a state of waiting to receive next image data.

If the reception confirmation signal can be received by the wireless reception section 5 during the period from the time t13 to the time t14, the control section 8 of the in-vivo information acquisition apparatus 1 obtains a judgment result that the condition of communication between the in-vivo information acquisition apparatus 1 and the terminal apparatus 11 is good, that is, the image data F1 acquired by the image pickup section 3 has been stored into the terminal apparatus 11.

Next, the control section 8 of the in-vivo information acquisition apparatus 1 performs control for causing the image pickup section 3 to pick up an image of the object in the body cavity of the subject 201 to acquire image data F2 during a period from time t15 to time t16. Furthermore, the control section 8 of the in-vivo information acquisition apparatus 1 performs control for causing the image data F2 acquired by the image pickup section 3 to be transmitted from the wireless transmission section 4 to the terminal apparatus 11 during a period from the time t16 to time t17, on the basis of the judgment result that the image data F1 has been stored into the terminal apparatus 11.

On the other hand, if a wireless signal which includes the image data F2 cannot be received by the reception antenna 13 after the time t14, the control section 15 of the terminal apparatus 11 maintains the state of waiting to receive next image data without performing generation and transmission of a reception confirmation signal.

If a reception confirmation signal cannot be received by the wireless reception section 5 during a period after completion of transmission of the image data F2 until time t18 before start of acquisition of next image data F3, the control section 8 of the in-vivo information acquisition apparatus 1 obtains a judgment result that the condition of communication between the in-vivo information acquisition apparatus 1 and the terminal apparatus 11 is bad, that is, the image data F2 acquired by the image pickup section 3 has not been stored into the terminal apparatus 11.

Next, the control section 8 of the in-vivo information acquisition apparatus 1 performs control for causing the image pickup section 3 to pick up an image of the object in the body cavity of the subject 201 to acquire the image data F3 during a period from time t19 to time t20. Furthermore, the control section 8 of the in-vivo information acquisition apparatus 1 performs control for causing the image data F3 acquired by the image pickup section 3 to be transmitted from the wireless transmission section 4 to the terminal apparatus 11 at the same time, causing the image data F3 to be stored into the storage section 6 during a period from the time t20 to time t21, on the basis of the judgment result that the image data F2 has not been stored into the terminal apparatus 11.

After that, as the control section 8 of the in-vivo information acquisition apparatus 1 continues to receive a judgment result that image data has not been stored into the terminal apparatus 11 because a reception confirmation signal cannot be received by the wireless reception section 5, the control section 8 of the in-vivo information acquisition apparatus 1 performs control so as to cause image data F4, F5, ..., F(N-1) temporally sequentially acquired by the image pickup section 3 to be transmitted from the wireless transmission section 4 to the terminal apparatus 11 at the same time, causing the image data F4, F5, ..., F(N-1) to be stored into the storage section 6. That is, if the condition of communication between the in-vivo information acquisition apparatus 1 and the terminal apparatus 11 is bad, the control section 8 of the in-vivo information acquisition apparatus 1 operates so as to cause image data acquired by the image pickup section 3 to be stored into the storage section 6.

Then, the control section 8 of the in-vivo information acquisition apparatus 1 performs control for causing image data FN acquired by the image pickup section 3 to be transmitted from the wireless transmission section 4 to the terminal apparatus 11 as well as causing the image data FN to be stored into the storage section 6 during a period from time t22 to time t23, on the basis of a judgment result that the image data F(N-1) has not been stored into the terminal apparatus 11.

If a wireless signal which includes the image data FN can be received by the reception antenna 13 during the period from the time t22 to the time t23, the control section 15 of the terminal apparatus 11 causes the image data FN to be stored into the storage section 14. Furthermore, the control section 15 of the terminal apparatus 11 performs control for causing a reception confirmation signal indicating that the wireless signal which includes the image data FN could be received to be generated and transmitted from the transmission antenna 12 to the in-vivo information acquisition apparatus 1 during a period from the time t23 to time t24. Then immediately after transmission of the reception confirmation signal indicating that the wireless signal which includes the image data FN could be received is completed, the control section 15 of the terminal apparatus 11 transitions to the state of waiting to receive next image data.

If the reception confirmation signal can be received by the wireless reception section 5 during the period from the time t23 to the time t24, the control section 8 of the in-vivo information acquisition apparatus 1 obtains a judgment result that condition of communication between the in-vivo information acquisition apparatus 1 and the terminal apparatus 11 is good, that is, the image data FN acquired by the image pickup section 3 has been stored into the terminal apparatus 11.

Then, the control section 8 of the in-vivo information acquisition apparatus 1 performs control for causing the image data F3 stored in the storage section 6 to be read out and transmitted from the wireless transmission section 4 to the terminal apparatus 11 during a period from time t25 to time t26, which is a period included in anywhere between a timing of receiving the reception confirmation signal for the image data FN and a timing of starting acquisition of next image data F(N+1), on the basis of the judgment result that the image data FN has been stored into the terminal apparatus 11.

If a wireless signal which includes the image data F3 can be received by the reception antenna 13 during the period from the time t25 to the time t26, the control section 15 of the terminal apparatus 11 causes the image data F3 to be stored into the storage section 14. Furthermore, the control section 15 of the terminal apparatus 11 performs control for causing a reception confirmation signal indicating that the wireless signal which includes the image data F3 could be received to be generated and transmitted from the transmission antenna 12 to the in-vivo information acquisition apparatus 1 during a period from the time t26 to time t27. Then, immediately after transmission of the reception confirmation signal indicating that the wireless signal which includes the image data F3 could be received is completed, the control section 15 of the terminal apparatus 11 transitions to the state of waiting to receive next image data.

The control section 8 of the in-vivo information acquisition apparatus 1 obtains a judgment result that the image data F3 read from the storage section 6 has been stored into the terminal apparatus 11, on the basis of the fact that the reception confirmation signal could be received by the wireless reception section 5 during the period from the time t26 to the time t27. Then, the control section 8 of the in-vivo information acquisition apparatus 1 performs control for causing the image pickup section 3 to pick up an image of the object in the body cavity of the subject 201 to acquire the image data F(N+1) during a period from time t27 to time t28 immediately after acquisition of the judgment result that the image data F3 has been stored into the terminal apparatus 11. Furthermore, the control section 8 of the in-vivo information acquisition apparatus 1 performs control for causing the image data F(N+1) acquired by the image pickup section 3 to be transmitted from the wireless transmission section 4 to the terminal apparatus 11 during a period from the time t28 to time t29.

After that, as the control section 8 of the in-vivo information acquisition apparatus 1 continues to receive a judgment result that image data has been stored into the terminal apparatus 11 because a reception confirmation signal could be received by the wireless reception section 5, the control section 8 of the in-vivo information acquisition apparatus 1 performs control for causing the image data F4, F5, ..., F(N-1) stored in the storage section 6 to be sequentially read out as well as causing the image data F4, F5, ..., F(N-1) read out from the storage section 6 and image data newly acquired by the image pickup section 3 to be alternately transmitted from the wireless transmission section 4 to the terminal apparatus 11. That is, when the condition of communication between the in-vivo information acquisition apparatus 1 and the terminal apparatus 11 is improved and becomes good, the control section 8 of the in-vivo information acquisition apparatus 1 operates so as to cause groups of image data stored into the storage section 6 during a period during which the condition of communication between the in-vivo information acquisition apparatus 1 and the terminal apparatus 11 is bad to be sequentially read out and transmitted from the wireless transmission section 4 to the terminal apparatus 11.

Note that the control section 15 of the terminal apparatus 11 of the present embodiment is not limited to one that operates so as to cause one reception confirmation signal to be generated and transmitted when a wireless signal which includes image data can be received once. For example, the control section 15 may operate so as to cause one reception confirmation signal to be generated and transmitted when a wireless signal which includes image data can be received a predetermined number of times equal to twice or more.

Furthermore, the control section 8 of the in-vivo information acquisition apparatus 1 of the present embodiment is not limited to one that performs control related to transmission of image data each time the image pickup section 3 acquires image data once during a period during which a reception confirmation signal cannot be received. For example, the control section 8 may perform the control related to transmission of image data each time the image pickup section 3 acquires image data a predetermined number of times equal to twice or more.

Furthermore, the control section 8 of the in-vivo information acquisition apparatus 1 of the present embodiment may delete image data accumulated in the storage section 6 all together when it can be confirmed that all image data acquired by the image pickup section 3 has been stored into the terminal apparatus 11. Alternatively, the control section 8 of the in-vivo information acquisition apparatus 1 of the present embodiment may divide a group of image data accumulated in the storage section 6 to sequentially delete the divided image data multiple times when it can be confirmed that image data acquired by the image pickup section 3 has been stored into the terminal apparatus 11.

As described above, the in-vivo information acquisition system 101 of the present embodiment has a configuration capable of using a wireless signal which includes image data obtained by actually picking up an image of an object to confirm whether the condition of communication between the in-vivo information acquisition apparatus 1 and the terminal apparatus 11 is good or bad. Therefore, according to the in-vivo information acquisition system 101 of the present embodiment, it is possible to simplify control related to communication operations between the in-vivo information acquisition apparatus 1 and the terminal apparatus 11 in comparison with conventional systems as well as reducing power consumed at the time of the communication operations between the in-vivo information acquisition apparatus 1 and the terminal apparatus 11 in comparison with the conventional systems.

### (Second Embodiment)

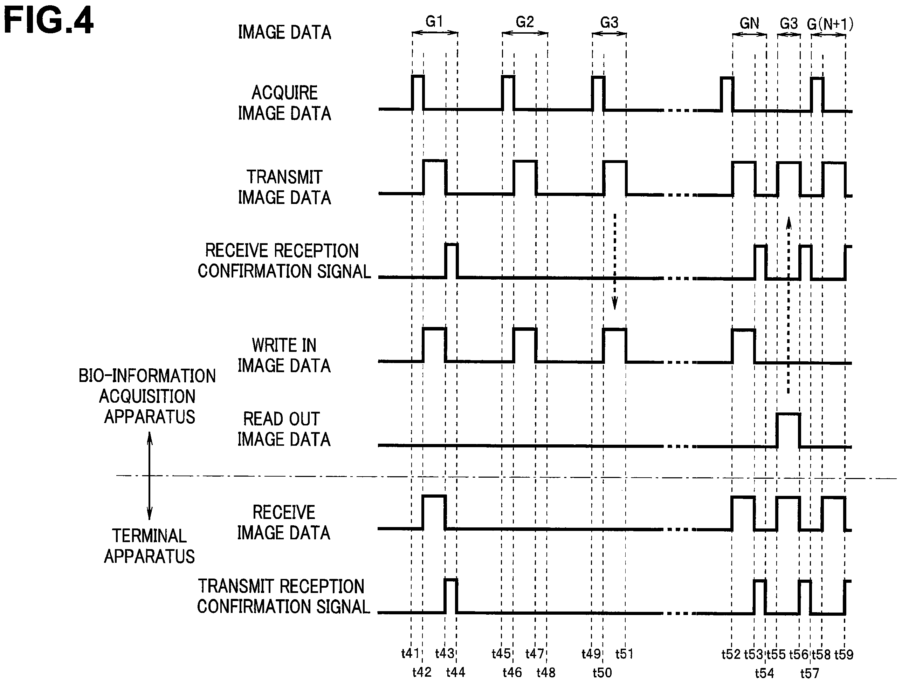

Fig. 4 relates to a second embodiment of the present invention. Note that an in-vivo information acquisition system in the present embodiment has a configuration substantially similar to that of the in-vivo information acquisition system 101 in the first embodiment. Therefore, in the present embodiment, description will be made mainly on parts which perform operations different from those of the in-vivo information acquisition system 101 in the first embodiment.

Here, specific operations of an in-vivo information acquisition apparatus 1 and a terminal apparatus 11 in the present embodiment will be described with appropriate reference to a timing chart in Fig. 4. Fig. 4 is a timing chart for illustrating operations of the in-vivo information acquisition apparatus and the terminal apparatus in the second embodiment.

First, at arbitrary timing before time t41, each of power sources for the in-vivo information acquisition apparatus 1 and the terminal apparatus 11 is on, and each of the in-vivo information acquisition apparatus 1 and the terminal apparatus 11 is arranged so that the in-vivo information acquisition apparatus 1 and the terminal apparatus 11 have the positional relationship illustrated in Fig. 2.

After that, a control section 8 of the in-vivo information acquisition apparatus 1 performs control for causing an image pickup section 3 to pick up an image of an object in a body cavity of a subject 201 to acquire image data G1 during a period from the time t41 to time t42. Furthermore, the control section 8 of the in-vivo information acquisition apparatus 1 performs control for causing the image data G1 acquired by the image pickup section 3 to be transmitted from a wireless transmission section 4 to the terminal apparatus 11, at the same time, performs control for causing the image data G1 to be stored into a storage section 6 during a period from the time t42 to time t43.

If a wireless signal which includes the image data G1 can be received by a reception antenna 13 during the period from the time t42 to the time t43, a control section 15 of the terminal apparatus 11 causes the image data G1 to be stored into a storage section 14. Furthermore, the control section 15 of the terminal apparatus 11 performs control for causing a reception confirmation signal indicating that the wireless signal which includes the image data G1 could be received to be generated and transmitted from a transmission antenna 12 to the in-vivo information acquisition apparatus 1 during a period from the time t43 to time t44. Then, immediately after transmission of the reception confirmation signal indicating that the wireless signal which includes the image data G1 could be received is completed, the control section 15 of the terminal apparatus 11 transitions to the state of waiting to receive next image data.

If the reception confirmation signal can be received by a wireless reception section 5 during the period from the time t43 to the time t44, the control section 8 of the in-vivo information acquisition apparatus 1 obtains a judgment result that condition of communication between the in-vivo information acquisition apparatus 1 and the terminal apparatus 11 is good, that is, the image data G1 acquired by the image pickup section 3 has been stored into the terminal apparatus 11 as well as giving identification information by which the judgment result can be identified, to the image data G1 stored in the storage section 6.

Next, the control section 8 of the in-vivo information acquisition apparatus 1 performs control for causing the image pickup section 3 to pick up an image of the object in the body cavity of the subject 201 to acquire image data G2 during a period from time t45 to time t46. Furthermore, the control section 8 of the in-vivo information acquisition apparatus 1 performs control for causing the image data G2 acquired by the image pickup section 3 to be transmitted from the wireless transmission section 4 to the terminal apparatus 11 , and at the same time, performs control for causing the image data G2 to be stored into the storage section 6 during a period from the time t46 to time t47.

On the other hand, if a wireless signal which includes the image data G2 cannot be received by the reception antenna 13 after the time t44, the control section 15 of the terminal apparatus 11 maintains the state of waiting to receive next image data without performing generation and transmission of a reception confirmation signal.

If a reception confirmation signal cannot be received by the wireless reception section 5 during a period after completion of transmission of the image data G2 until time t48 before start of acquisition of next image data G3, the control section 8 of the in-vivo information acquisition apparatus 1 obtains a judgment result that the condition of communication between the in-vivo information acquisition apparatus 1 and the terminal apparatus 11 is bad, that is, the image data G2 acquired by the image pickup section 3 has not been stored into the terminal apparatus 11 as well as giving identification information by which the judgment result can be identified, to the image data G2 stored in the storage section 6.

Next, the control section 8 of the in-vivo information acquisition apparatus 1 performs control for causing the image pickup section 3 to pick up an image of the object in the body cavity of the subject 201 to acquire image data G3 during a period from time t49 to time t50. Furthermore, the control section 8 of the in-vivo information acquisition apparatus 1 performs control for causing the image data G3 acquired by the image pickup section 3 to be transmitted from the wireless transmission section 4 to the terminal apparatus 11 and at the same time, causing the image data G3 to be stored into the storage section 6 during a period from the time t50 to time t51.

After that, as the control section 8 of the in-vivo information acquisition apparatus 1 continues to receive a judgment result that image data has not been stored into the terminal apparatus 11 because a reception confirmation signal cannot be received by the wireless reception section 5, the control section 8 of the in-vivo information acquisition apparatus 1 causes image data G4, G5, ..., G(N-1) temporally sequentially acquired by the image pickup section 3 to be transmitted from the wireless transmission section 4 to the terminal apparatus 11 as well as giving identification information by which the judgment result can be identified, to the image data G3, G4, G5, ..., G(N-1) stored in the storage section 6.

Then, the control section 8 of the in-vivo information acquisition apparatus 1 performs control for causing the image data GN acquired by the image pickup section 3 to be transmitted from the wireless transmission section 4 to the terminal apparatus 11 and at the same time, causing the image data GN to be stored into the storage section 6 during a period from time t52 to time t53.

If a wireless signal which includes the image data GN can be received by the reception antenna 13 during the period from the time t52 to the time t53, the control section 15 of the terminal apparatus 11 causes the image data GN to be stored into the storage section 14. Furthermore, the control section 15 of the terminal apparatus 11 performs control for causing a reception confirmation signal indicating that the wireless signal which includes the image data GN could be received to be generated and transmitted from the transmission antenna 12 to the in-vivo information acquisition apparatus 1 during a period from the time t53 to time t54. Then immediately after transmission of the reception confirmation signal indicating that the wireless signal which includes the image data GN could be received is completed, the control section 15 of the terminal apparatus 11 transitions to the state of waiting to receive next image data.

If the reception confirmation signal can be received by the wireless reception section 5 during the period from the time t53 to the time t54, the control section 8 of the in-vivo information acquisition apparatus 1 obtains a judgment result that the condition of communication between the in-vivo information acquisition apparatus 1 and the terminal apparatus 11 is good, that is, the image data GN acquired by the image pickup section 3 has been stored into the terminal apparatus 11 as well as giving identification information by which the judgment result can be identified, to the image data GN stored in the storage section 6.

Then, the control section 8 of the in-vivo information acquisition apparatus 1 performs control for causing the image data G3 to which information of not being stored in the terminal apparatus 11 is given to be read out from the storage section 6 and transmitted from the wireless transmission section 4 to the terminal apparatus 11 during a period from time t55 to time t56, which is a period included in anywhere between a timing of receiving the reception confirmation signal for the image data GN and a timing of starting acquisition of next image data G(N+1), on the basis of the judgment result that the image data GN has been stored into the terminal apparatus 11.

If a wireless signal which includes the image data G3 can be received by the reception antenna 13 during the period from the time t55 to the time t56, the control section 15 of the terminal apparatus 11 causes the image data G3 to be stored into the storage section 14. Furthermore, the control section 15 of the terminal apparatus 11 performs control for causing a reception confirmation signal indicating that the wireless signal which includes the image data G3 could be received to be generated and transmitted from the transmission antenna 12 to the in-vivo information acquisition apparatus 1 during a period from the time t56 to time t57. Then immediately after transmission of the reception confirmation signal indicating that the wireless signal which includes the image data G3 could be received is completed, the control section 15 of the terminal apparatus 11 transitions to the state of waiting to receive next image data.

The control section 8 of the in-vivo information acquisition apparatus 1 obtains a judgment result that the image data G3 has been stored into the terminal apparatus 11 as well as updating the identification information given to the image data G3 in the storage section 6, on the basis of the fact that the reception confirmation signal could be received by the wireless reception section 5 during the period from the time t56 to the time t57. Then, the control section 8 of the in-vivo information acquisition apparatus 1 performs control for causing the image pickup section 3 to pick up an image of the object in the body cavity of the subject 201 to acquire the image data G(N+1) during a period from time t57 to time t58 immediately after acquisition of the judgment result that the image data G3 has been stored into the terminal apparatus 11. Furthermore, the control section 8 of the in-vivo information acquisition apparatus 1 performs control for causing the image data G(N+1) acquired by the image pickup section 3 to be transmitted from the wireless transmission section 4 to the terminal apparatus 11 during a period from the time t58 to time t59.

After that, as the control section 8 of the in-vivo information acquisition apparatus 1 continues to receive a judgment result that image data has been stored into the terminal apparatus 11 because a reception confirmation signal could be received by the wireless reception section 5, the control section 8 of the in-vivo information acquisition apparatus 1 performs control for causing the image data G4, G5, ..., G(N-1) to which the information of not being stored in the terminal apparatus 11 is given to be sequentially read out from the storage section 6 as well as causing the image data G4, G5, ..., G(N-1) read out from the storage section 6 and image data newly acquired by the image pickup section 3 to be alternately transmitted from the wireless transmission section 4 to the terminal apparatus 11. That is, when the condition of communication between the in-vivo information acquisition apparatus 1 and the terminal apparatus 11 is improved and becomes good, the control section 8 of the in-vivo information acquisition apparatus 1 operates so as to cause groups of image data stored into the storage section 6 during a period during which the condition of communication between the in-vivo information acquisition apparatus 1 and the terminal apparatus 11 is bad to be sequentially read out and transmitted from the wireless transmission section 4 to the terminal apparatus 11.

Note that the control section 8 of the in-vivo information acquisition apparatus 1 of the present embodiment may delete a group of image data to which information of having been stored into the terminal apparatus 11 is given all together from the storage section 6 when it can be confirmed that all image data acquired by the image pickup section 3 has been stored into the terminal apparatus 11. Alternatively, the control section 8 of the in-vivo information acquisition apparatus 1 of the present embodiment may divide the group of image data to which the information of having been stored into the terminal apparatus 11 is given and sequentially delete the divided image data from the storage section 6 multiple times.

As described above, the in-vivo information acquisition system 101 of the present embodiment has a configuration capable of using a wireless signal which includes image data obtained by actually picking up an image of an object to confirm whether the condition of communication between the in-vivo information acquisition apparatus 1 and the terminal apparatus 11 is good or bad. Therefore, according to the in-vivo information acquisition system 101 of the present embodiment, it is possible to simplify control related to communication operations between the in-vivo information acquisition apparatus 1 and the terminal apparatus 11 in comparison with conventional systems as well as reducing power consumed at the time of the communication operations between the in-vivo information acquisition apparatus 1 and the terminal apparatus 11 in comparison with the conventional systems.

Furthermore, the in-vivo information acquisition system 101 of the present embodiment has a configuration capable of performing transmission of image data acquired by the image pickup section 3 to the terminal apparatus 11 and storage of the image data into the storage section 6 simultaneously or substantially simultaneously. Therefore, according to the in-vivo information acquisition system 101 of the present embodiment, it is possible to prevent missing of image data acquired by the image pickup section 3, for example, even under such an environment that the condition of communication between the in-vivo information acquisition apparatus 1 and the terminal apparatus 11 deteriorates intermittently.

Note that the present invention is not limited to each embodiment described above, and it goes without saying that various alterations and applications are possible.

## Claims

1. An in-vivo information acquisition system (101) comprising an in-vivo information acquisition apparatus (1) provided with a configuration capable of transmitting a wireless signal which includes in-vivo information about an inside of a subject (201) acquired by an in-vivo information acquisition section (2, 3) to an outside of the subject (201), and a terminal apparatus (11) provided with a configuration capable of receiving the wireless signal transmitted from the in-vivo information acquisition apparatus (1) outside the subject (201), wherein
the terminal apparatus (11) comprises a reception confirmation signal transmission section (12, 15) generating a reception confirmation signal and transmitting the reception confirmation signal to the in-vivo information acquisition apparatus (1) when the wireless signal which includes the in-vivo information about the subject (201) can be received; and
the in-vivo information acquisition apparatus (1) comprises a storage section (6), and a control section (8) causing the in-vivo information about the subject (201) to be stored into the storage section (6) at least during a period during which the reception confirmation signal transmitted from the terminal apparatus (11) cannot be received,
**characterized in that**
the control section (8) is adapted to cause at the same time the wireless signal, which includes the same in-vivo information about the subject (201) as being stored in the storage section (6), to be transmitted to the outside of the subject (201) at least during the period during which the reception confirmation signal transmitted from the terminal apparatus (11) cannot be received.

2. The in-vivo information acquisition system (101) according to claim 1, wherein the control section (8) causes groups of in-vivo information stored in the storage section (6) to be sequentially read out and wirelessly transmitted to the terminal apparatus (11) after detecting transition from the period during which the reception confirmation signal transmitted from the terminal apparatus (11) cannot be received to a period during which the reception confirmation signal can be received.

3. The in-vivo information acquisition system (101) according to claim 1, wherein the control section (8) causes the wireless signals that include the in-vivo information acquired by the in-vivo information acquisition section (2, 3) to be sequentially transmitted to the outside of the subject (201), causes the same in-vivo information as included in the wireless signals transmitted to the outside of the subject (201) to be sequentially stored into the storage section (6), and further gives identification information capable of identifying whether in-vivo information is in-vivo information acquired during the period during which the reception confirmation signal transmitted from the terminal apparatus (11) cannot be received, to each in-vivo information stored in the storage section (6).

4. The in-vivo information acquisition system (101) according to claim 3, wherein the control section (8) causes groups of in-vivo information acquired during the period during which the reception confirmation signal transmitted from the terminal apparatus (11) cannot be received to be sequentially read out from the storage section (6) and wirelessly transmitted to the terminal apparatus (11) on the basis of the identification information after detecting transition from the period during which the reception confirmation signal transmitted from the terminal apparatus (11) cannot be received to a period during which the reception confirmation signal can be received.

5. A method for controlling an in-vivo information acquisition system (101) comprising an in-vivo information acquisition apparatus (1) provided with a configuration capable of transmitting a wireless signal which includes in-vivo information about an inside of a subject (201) acquired by an in-vivo information acquisition section (2, 3) to an outside of the subject (201), and a terminal apparatus (11) provided with a configuration capable of receiving the wireless signal transmitted from the in-vivo information acquisition apparatus (1) outside the subject (201), the method comprising the steps of:
a reception confirmation signal being generated and transmitted to the in-vivo information acquisition apparatus (1) by an operation of a reception confirmation signal transmission section (12, 15) of the terminal apparatus (11) when the wireless signal which includes the in-vivo information about the subject (201) is received;
the in-vivo information about the subject (201) being stored into a storage section (6) by an operation of the control section (8) at least during a period during which the reception confirmation signal transmitted from the terminal apparatus (11) cannot be received,
**characterized by**
the wireless signal, which includes the same in-vivo information about the subject (201) as being stored into the storage section (6), being transmitted to the outside of the subject (201) by an operation of the control section (8) of the in-vivo information acquisition apparatus (1) at least during the period during which the reception confirmation signal transmitted from the terminal apparatus (11) cannot be received.

6. The in-vivo information acquisition system (101) control method according to claim 5, further comprising a step of groups of in-vivo information stored in the storage section (6) being sequentially read out and wirelessly transmitted to the terminal apparatus (11) by an operation of the control section (8) after transition from the period during which the reception confirmation signal transmitted from the terminal apparatus (11) cannot be received to a period during which the reception confirmation signal can be received is detected.

7. The in-vivo information acquisition system (101) control method according to claim 5, further comprising the steps of:
the wireless signals that include the in-vivo information acquired by the in-vivo information acquisition section (2, 3) being sequentially transmitted to the outside of the subject (201) by an operation of the control section (8);
the same in-vivo information as included in the wireless signals transmitted to the outside of the subject (201) being sequentially stored into the storage section (6) by an operation of the control section (8); and
identification information capable of identifying whether in-vivo information is in-vivo information acquired during the period during which the reception confirmation signal transmitted from the terminal apparatus (11) cannot be received being given to each in-vivo information stored in the storage section (6) by an operation of the control section (8).

8. The in-vivo information acquisition system (101) control method according to claim 7, further comprising a step of groups of in-vivo information acquired during the period during which the reception confirmation signal transmitted from the terminal apparatus (11) cannot be received being sequentially read out from the storage section (6) and wirelessly transmitted to the terminal apparatus (11) by an operation of the control section (8) based on the identification information after transition from the period during which the reception confirmation signal transmitted from the terminal apparatus (11) cannot be received to a period during which the reception confirmation signal can be received is detected.

## Patentansprüche

1. In-vivo-Informationsbeschaffungssystem (101), das eine In-vivo-Informationsbeschaffungsvorrichtung (1), die eine Konfiguration aufweist, die in der Lage ist, ein drahtloses Signal, das In-vivo-Informationen über ein Inneres eines Subjektes (201), die von einem In-vivo-Informationsbeschaffungsabschnitt (2, 3) beschafft werden, enthält, zur Außenseite des Subjektes (201) zu übertragen, und eine Terminalvorrichtung (11) aufweist, die eine Konfiguration aufweist, die in der Lage ist, das drahtlose Signal, das von der In-vivo-Informationsbeschaffungsvorrichtung (1) übertragen wird, außerhalb des Subjektes (201) zu empfangen, wobei
die Terminalvorrichtung (11) einen Empfangsbestätigungssignalübertragungsabschnitt (12, 15) aufweist, der ein Empfangsbestätigungssignal erzeugt und das Empfangsbestätigungssignal an die In-vivo-Informationsbeschaffungsvorrichtung (1) überträgt, wenn das drahtlose Signal, das die In-vivo-Informationen über das Subjekt (201) enthält, empfangen werden kann; und
die In-vivo-Informationsbeschaffungsvorrichtung (1) einen Speicherabschnitt (6) und einen Steuerabschnitt (8) aufweist, der bewirkt, dass die In-vivo-Informationen über das Subjekt (201) in dem Speicherabschnitt (6) zumindest während einer Periode, während der das Empfangsbestätigungssignal, das von der Terminalvorrichtung (11) übertragen wird, nicht empfangen werden kann, gespeichert werden,
**dadurch gekennzeichnet, dass**
der Steuerabschnitt (8) ausgelegt ist, gleichzeitig zu bewirken, dass das drahtlose Signal, das dieselben In-vivo-Informationen über das Subjekt (201) wie sie in dem Speicherabschnitt (6) gespeichert sind, enthält, zur Außenseite des Subjektes (201) zumindest während der Periode, während der das Empfangsbestätigungssignal, das von der Terminalvorrichtung (11) übertragen wird, nicht empfangen werden kann, übertragen wird.

2. In-vivo-Informationsbeschaffungssystem (101) nach Anspruch 1, wobei der Steuerabschnitt (8) bewirkt, dass Gruppen von In-vivo-Informationen, die in dem Speicherabschnitt (6) gespeichert sind, aufeinanderfolgend ausgelesen und drahtlos an die Terminalvorrichtung (11) nach Erfassen eines Übergangs von der Periode, während der das Empfangsbestätigungssignal, das von der Terminalvorrichtung (11) übertragen wird, nicht empfangen werden kann, zu einer Periode, während der das Empfangsbestätigungssignal empfangen werden kann, übertragen werden.

3. In-vivo-Informationsbeschaffungssystem (101) nach Anspruch 1, wobei der Steuerabschnitt (8) bewirkt, dass die drahtlosen Signale, die die In-vivo-Informationen enthalten, die durch den In-vivo-Informationsbeschaffungsabschnitt (2, 3) beschafft werden, aufeinanderfolgend zur Außenseite des Subjektes (201) übertragen werden, bewirkt, dass dieselben In-vivo-Informationen wie sie in den drahtlosen Signalen enthalten sind, die zur Außenseite des Subjektes (201) übertragen werden, aufeinanderfolgend in dem Speicherabschnitt (6) gespeichert werden, und außerdem Identifizierungsinformationen, die in der Lage sind, zu identifizieren, ob In-vivo-Informationen In-vivo-Informationen sind, die während der Periode, während der das Empfangsbestätigungs-signal, das von der Terminalvorrichtung (11) übertragen wird, nicht empfangen werden kann, beschafft werden, für jede In-vivo-Information, die in dem Speicherabschnitt (6) gespeichert ist, vergibt.

4. In-vivo-Informationsbeschaffungssystem (101) nach Anspruch 3, wobei der Steuerabschnitt (8) bewirkt, dass Gruppen von In-vivo-Informationen, die während der Periode beschafft werden, während der das Empfangsbestätigungssignal, das von der Terminalvorrichtung (11) übertragen wird, nicht empfangen werden kann, aufeinanderfolgend aus dem Speicherabschnitt (6) ausgelesen und drahtlos an die Terminalvorrichtung (11) auf der Grundlage der Identifizierungsinformationen nach einem Erfassen eines Übergangs von der Periode, während der das Empfangsbestätigungssignal, das von der Terminalvorrichtung (11) übertragen wird, nicht empfangen werden kann, zu einer Periode, während der das Empfangsbestätigungssignal empfangen werden kann, übertragen werden.

5. Verfahren zum Steuern eines In-vivo-Informationsbeschaffungssystems (101), das eine In-vivo-Informationsbeschaffungsvorrichtung (1), die eine Konfiguration aufweist, die in der Lage ist, ein drahtloses Signal, das in In-vivo-Informationen über ein Inneres eines Subjektes (201), die von einem In-vivo-Informationsbeschaffungsabschnitt (2, 3) beschafft werden, enthält, zur Außenseite des Subjektes (201) zu übertragen, und eine Terminalvorrichtung (11) aufweist, die eine Konfiguration aufweist, die in der Lage ist, das drahtlose Signal, das von der In-vivo-Informationsbeschaffungsvorrichtung (1) übertragen wird, außerhalb des Subjektes (201) zu empfangen, wobei das Verfahren die folgenden Schritte aufweist:
Erzeugen und Übertragen eines Empfangsbestätigungssignals durch einen Betrieb eines Empfangsbestätigungssignalübertragungsabschnitts (12, 15) der Terminalvorrichtung (11), wenn das drahtlose Signal, das die In-vivo-Informationen über das Subjekt (201) enthält, empfangen wird;
Speichern der In-vivo-Informationen über das Subjekt (201) in einem Speicherabschnitt (6) durch einen Betrieb des Steuerabschnitts (8) zumindest während einer Periode, während der das Empfangsbestätigungssignal, das von der Terminalvorrichtung (11) übertragen wird, nicht empfangen werden kann,
**gekennzeichnet durch**
Übertragen des drahtlosen Signals, das dieselben In-vivo-Informationen über das Subjekt (201) wie sie in dem Speicherabschnitt (6) gespeichert sind enthält, zur Außenseite des Subjektes (201) **durch** einen Betrieb des Steuerabschnitts (8) der In-vivo-Informationsbeschaffungsvorrichtung (1) zumindest während der Periode, während der das Empfangsbestätigungssignal, das von der Terminalvorrichtung (11) übertragen wird, nicht empfangen werden kann.

6. Verfahren zum Steuern eines In-vivo-Informationsbeschaffungssystems (1) nach Anspruch 5, das außerdem einen Schritt zum aufeinanderfolgenden Auslesen und drahtlosen Übertragen von Gruppen von In-vivo-Informationen, die in dem Speicherabschnitt (6) gespeichert sind, an die Terminalvorrichtung (11) durch einen Betrieb des Steuerabschnitts (8) nach einem Erfassen eines Übergangs von der Periode, während der das Empfangsbestätigungssignal von der Terminalvorrichtung (11) nicht empfangen werden kann, zu einer Periode, während der das Empfangsbestätigungssignal empfangen werden kann, aufweist.

7. Verfahren zum Steuern eines In-vivo-Informationsbeschaffungssystems (101) nach Anspruch 5, das außerdem die folgenden Schritte aufweist:
aufeinanderfolgendes Übertragen der drahtlosen Signale, die die In-vivo-Informationen enthalten, die von dem In-vivo-Informationsbeschaffungsabschnitt (2, 3) beschafft werden, zur Außenseite des Subjektes (201) durch einen Betrieb des Steuerabschnitts (8);
aufeinanderfolgendes Speichern derselben In-vivo-Informationen wie sie in den drahtlosen Signalen enthalten sind, die zur Außenseite des Subjektes (201) übertragen werden, in dem Speicherabschnitt (6) durch einen Betrieb des Steuerabschnitts (8); und
Vergeben von Identifizierungsinformationen, die in der Lage sind, zu identifizieren, ob In-vivo-Informationen In-vivo-Informationen sind, die während der Periode beschafft werden, während der das Empfangsbestätigungssignal, das von der Terminalvorrichtung (11) übertragen wird, nicht empfangen werden kann, für jede In-vivo-Information, die in dem Speicherabschnitt (6) gespeichert ist, durch einen Betrieb des Steuerabschnitts (8).

8. Verfahren zum Steuern eines In-vivo-Informationsbeschaffungssystems (101) nach Anspruch 7, das außerdem einen Schritt zum aufeinanderfolgenden Auslesen von Gruppen von In-vivo-Informationen, die während der Periode beschafft werden, während der das Empfangsbestätigungssignal, das von der Terminalvorrichtung (11) übertragen wird, nicht empfangen werden kann, aus dem Speicherabschnitt (6) und drahtlosen Übertragen derselben an die Terminalvorrichtung (11) durch einen Betrieb des Steuerabschnitts (8) auf der Grundlage der Identifizierungsinformationen nach einem Erfassen eines Übergangs von der Periode, während der das Empfangsbestätigungssignal, das von der Terminalvorrichtung (11) übertragen wird, nicht empfangen werden kann, zu einer Periode, während der das Empfangsbestätigungssignal empfangen werden kann.

## Revendications

1. Système d'acquisition d'informations in-vivo (101) comprenant un appareil d'acquisition d'informations in-vivo (1) pourvu d'une configuration capable de transmettre un signal sans fil qui inclut des informations in-vivo concernant un intérieur d'un sujet (201) acquises par une section d'acquisition d'informations in-vivo (2, 3) à un extérieur du sujet (201), et un appareil terminal (11) pourvu d'une configuration capable de recevoir le signal sans fil transmis depuis l'appareil d'acquisition d'informations in-vivo (1) à l'extérieur du sujet (201), dans lequel
l'appareil terminal (11) comprend une section de transmission de signal de confirmation de réception (12, 15) générant un signal de confirmation de réception et transmettant le signal de confirmation de réception à l'appareil d'acquisition d'informations in-vivo (1) quand le signal sans fil qui inclut les informations in-vivo concernant le sujet (201) peut être reçu ; et
l'appareil d'acquisition d'informations in-vivo (1) comprend une section de stockage (6), et une section de commande (8) amenant les informations in-vivo concernant le sujet (201) à être stockées dans la section de stockage (6) au moins durant une période pendant laquelle le signal de confirmation de réception transmis depuis l'appareil terminal (11) ne peut pas être reçu,
**caractérisé en ce que**
la section de commande (8) est adaptée pour amener en même temps le signal sans fil, qui inclut ces mêmes informations in-vivo concernant le sujet (201) stockées dans la section de stockage (6), à être transmis à l'extérieur du sujet (201) au moins durant la période pendant laquelle le signal de confirmation de réception transmis depuis l'appareil terminal (11) ne peut pas être reçu.

2. Système d'acquisition d'informations in-vivo (101) selon la revendication 1, dans lequel la section de commande (8) amène des groupes d'informations in-vivo stockées dans la section de stockage (6) à être séquentiellement lus et transmis sans fil à l'appareil terminal (11) après détection d'une transition de la période durant laquelle le signal de confirmation de réception transmis depuis l'appareil terminal (11) ne peut pas être reçu à une période durant laquelle le signal de confirmation de réception peut être reçu.

3. Système d'acquisition d'informations in-vivo (101) selon la revendication 1, dans lequel la section de commande (8) amène les signaux sans fil qui incluent les d'informations in-vivo acquises par la section d'acquisition d'informations in-vivo (2, 3) à être séquentiellement transmis à l'extérieur du sujet (201), amène ces mêmes informations in-vivo incluses dans les signaux sans fil transmis à l'extérieur du sujet (201) à être séquentiellement stockées dans la section de stockage (6) et donne en outre des informations d'identification capables d'identifier si les informations in-vivo sont des informations in-vivo acquises durant la période pendant laquelle le signal de confirmation de réception transmis depuis l'appareil terminal (11) ne peut pas être reçu, à chaque information in-vivo stockée dans la section de stockage (6).

4. Système d'acquisition d'informations in-vivo (101) selon la revendication 3, dans lequel la section de commande (8) amène des groupes d'informations in-vivo acquis durant la période pendant laquelle le signal de confirmation de réception transmis depuis l'appareil terminal (11) ne peut pas être reçu à être séquentiellement lus depuis la section de stockage (6) et transmis sans fil à l'appareil terminal (11) sur la base des informations d'identification après détection d'une transition de la période durant laquelle le signal de confirmation de réception transmis depuis l'appareil terminal (11) ne peut pas être reçu à une période durant laquelle le signal de confirmation de réception peut être reçu.

5. Procédé de commande d'un système d'acquisition d'informations in-vivo (101) comprenant un appareil d'acquisition d'informations in-vivo (1) pourvu d'une configuration capable de transmettre un signal sans fil qui inclut des informations in-vivo concernant un intérieur d'un sujet (201) acquises par une section d'acquisition d'informations in-vivo (2, 3) à un extérieur du sujet (201), et un appareil terminal (11) pourvu d'une configuration capable de recevoir le signal sans fil transmis depuis l'appareil d'acquisition d'informations in-vivo (1) à l'extérieur du sujet (201), le procédé comprenant les étapes suivantes :
un signal de confirmation de réception étant généré et transmis à l'appareil d'acquisition d'informations in-vivo (1) par une opération d'une section de transmission de signal de confirmation de réception (12, 15) de l'appareil terminal (11) quand le signal sans fil qui inclut les informations in-vivo concernant le sujet (201) peut être reçu ;
les informations in-vivo concernant le sujet (201) étant stockées dans une section de stockage (6) par une opération de la section de commande (8) au moins durant une période pendant laquelle le signal de confirmation de réception transmis depuis l'appareil terminal (11) ne peut pas être reçu,
**caractérisé en ce que**
le signal sans fil, qui inclut ces mêmes informations in-vivo concernant le sujet (201) stockées dans la section de stockage (6), étant transmis à l'extérieur du sujet (201) par une opération de la section de commande (8) de l'appareil d'acquisition d'informations in-vivo (1) au moins durant la période pendant laquelle le signal de confirmation de réception transmis depuis l'appareil terminal (11) ne peut pas être reçu.

6. Procédé de commande d'un système d'acquisition d'informations in-vivo (101) selon la revendication 5, comprenant en outre une étape de groupes d'informations in-vivo stockées dans la section de stockage (6) étant séquentiellement lus et transmis sans fil à l'appareil terminal (11) par une opération de la section de commande (8) après qu'une transition de la période durant laquelle le signal de confirmation de réception transmis depuis l'appareil terminal (11) ne peut pas être reçu à une période durant laquelle le signal de confirmation de réception peut être reçu a été détectée.

7. Procédé de commande d'un système d'acquisition d'informations in-vivo (101) selon la revendication 5, comprenant en outre une étape de :
les signaux sans fil qui incluent les informations in-vivo acquises par la section d'acquisition d'informations in-vivo (2, 3) étant séquentiellement transmis à l'extérieur du sujet (201) par une opération de la section de commande (8) ;
ces mêmes informations in-vivo incluses dans les signaux sans fil transmis à l'extérieur du sujet (201) étant séquentiellement stockées dans la section de stockage (6) par une opération de la section de commande (8) ; et
des informations d'identification capables d'identifier si les informations in-vivo sont des informations in-vivo acquises durant la période pendant laquelle le signal de confirmation de réception transmis depuis l'appareil terminal (11) ne peut pas être reçu étant données à chaque information in-vivo stockée dans la section de stockage (6) par une opération de la section de commande (8).

8. Procédé de commande d'un système d'acquisition d'informations in-vivo (101) selon la revendication 7, comprenant en outre une étape de groupes d'informations in-vivo acquis durant la période pendant laquelle le signal de confirmation de réception transmis depuis l'appareil terminal (11) ne peut pas être reçu étant séquentiellement lus depuis la section de stockage (6) et transmis sans fil à l'appareil terminal (11) par une opération de la section de commande (8) sur la base des informations d'identification après qu'une transition de la période durant laquelle le signal de confirmation de réception transmis depuis l'appareil terminal (11) ne peut pas être reçu à une période durant laquelle le signal de confirmation de réception peut être reçu a été détectée.
